# EUROPÄISCHE PATENTANMELDUNG

(11) **EP 1 110 512 A1**
(43) Veröffentlichungstag der Anmeldung: **27.06.2001**
(21) Anmeldenummer: 99811171.0
(22) Anmeldetag: 20.12.1999
(51) Int. Cl.: A61B 17/88, B25B 23/142

(54) **Medizinaltechnische Werkzeughaltevorrichtung mit Drehmomentbegrenzung**

(71) Anmelder: Sulzer Orthopedics Ltd., 6340 Baar (CH)
(72) Erfinder: Casutt, Simon, 9200 Gossau (CH)
(74) Vertreter: Sulzer Management AG

(57) **Zusammenfassung**

Die medizinaltechnische Werkzeughaltevorrichtung (2) mit Drehmomentbegrenzung, umfasst ein Gehäuse (5, 6) und einen darin um eine Drehachse (13) drehbar gelagerten Kugelmitnehmer (7), wobei der Kugelmitnehmer (7) ein in Richtung der Drehachse (13) verlaufendes Nabenteil (7g) sowie ein dazu radial verlaufendes Flanschteil (7h) umfasst, wobei im Flanschteil (7h) eine Mehrzahl in Umfangsrichtung beabstandete, im wesentlichen radial verlaufende Führungen (7e) zur Aufnahme einer Kugel (11) angeordnet sind, und wobei das Gehäuse (5, 6) eine im wesentlichen konzentrisch zur Drehachse (13) verlaufende, zur Drehachse (13) hin ausgerichtete Rollfläche (6b) mit in Umfangsrichtung beabstandet angeordneten Vertiefungen (6c) aufweist, und wobei ein Kraftumlenkelement (8) am Nabenteil (7g) in Richtung der Drehachse (13) verschiebbar und federbelastet gelagert ist, und wobei zwischen dem Flanschteil (7h) und dem Kraftumlenkelement (8) zumindest eine Kugel (11) angeordnet ist, und wobei die zum Flanschteil (7h) hin ausgerichtete Stirnseite (8a) des Kraftumlenkteils (8) derart ausgestaltet ist, dass die an der Stirnseite (8a) anliegende Kugel (11) eine radial wirkende Kraft erfährt, um die Kugel (11) gegen die Rollfläche (6b) oder in die Vertiefung (6c) zu drücken.

## Beschreibung

Die Erfindung betrifft eine medizinaltechnische Werkzeughaltevorrichtung mit Drehmomentbegrenzung gemäss dem Oberbegriff von Anspruch 1.

Aus der Mechanik ist eine Vielzahl von drehmomentübertragenden Kupplungen bekannt, welche nur ein begrenztes Drehmoment übertragen. Derartige Kupplungen werden auch als Überlastkupplungen bezeichnet.

Nachteilig an derartigen Kupplungen ist die Tatsache, dass sie für medizinaltechnische Anwendungen, beispielsweise eingebaut in Instrumente für chirurgische Eingriffe am Menschen, nicht geeignet sind, da sie nicht sterilisierbar sind, das maximal erzielbare Drehmoment relativ ungenau ist, und das maximal erzielbare Drehmoment längerfristig Änderungen unterworfen ist.

Es ist Aufgabe der vorliegenden Erfindung eine medizinaltechnische Werkzeughaltevorrichtung mit Drehmomentbegrenzung zu schaffen, welche sterilisierbar und wiederverwendbar ist, und zudem ein einstellbares, auch relativ hohes maximales Drehmoment aufweist, wobei das maximale Drehmoment auch längerfristig, beispielsweise nach mehrmaliger Wasserdampfsterilisation, reproduzierbar ist.

Diese Aufgabe wird gelöst mit einer medizinaltechnischen Werkzeughaltevorrichtung mit Drehmomentbegrenzung aufweisend die Merkmale von Anspruch 1. Die Unteransprüche 2 bis 9 betreffen weitere, vorteilhafte Ausgestaltungen von medizinaltechnischen Werkzeughaltevorrichtungen mit Drehmomentbegrenzung.

Die Aufgabe wird insbesondere gelöst mit einer medizinaltechnischen Werkzeughaltevorrichtung mit Drehmomentbegrenzung, umfassend ein Gehäuse und einen darin um eine Drehachse drehbar gelagerten Kugelmitnehmer, wobei der Kugelmitnehmer ein in Richtung der Drehachse verlaufendes Nabenteil sowie ein dazu radial verlaufendes Flanschteil umfasst, wobei im Flanschteil eine Mehrzahl in Umfangsrichtung beabstandete, im wesentlichen radial verlaufende Führungen zur Aufnahme einer Kugel angeordnet sind, und wobei das Gehäuse eine im wesentlichen konzentrisch zur Drehachse verlaufende, zur Drehachse hin ausgerichtete Rollfläche mit in Umfangsrichtung beabstandet angeordneten Vertiefungen aufweist, und wobei ein Kraftumlenkelement am Nabenteil in Richtung der Drehachse verschiebbar und federbelastet gelagert ist, und wobei zwischen dem Flanschteil und dem Kraftumlenkelement zumindest eine Kugel angeordnet ist, und wobei die zum Flanschteil hin ausgerichtete Stirnseite des Kraftumlenkteils derart ausgestaltet ist, dass die an der Stirnseite anliegende Kugel eine radial wirkende Kraft erfährt, um die Kugel gegen die Rollfläche oder in die Vertiefung zu drücken.

Die erfindungsgemässe medizinaltechnische Werkzeughaltevorrichtung ist in einer bevorzugten Ausführungsform auf der einen Seite mit einem Handgriff und auf der anderen Seite mit einem Werkzeug verbunden und bildet derart ein medizinaltechnisches, beispielsweise ein chirurgisches Instrument. In einer bevorzugten Ausgestaltung ist der Handgriff länglich ausgestaltet und definiert dabei eine Längsachse, um welche das Instrument mitsamt dem Werkzeug gedreht wird, wobei die zwischen Handgriff und Werkzeug angeordnete Werkzeughaltevorrichtung das maximal zu übertragende Drehmoment begrenzt. Für grössere Momente, beispielsweise grösser als 5 Nm, kann der Handgriff auch als T-Griff ausgestaltet sein.

Die erfindungsgemässe medizinaltechnische Werkzeughaltevorrichtung ermöglicht ein chirurgisches Instrument zusammengestellt aus einem Werkzeug, der Werkzeughaltevorrichtung sowie dem Handgriff zu bilden, wobei das chirurgische Instrument zum Reinigen in die drei genannten Teile zerlegbar ist und nach dem Reinigen wieder zusammengebaut werden kann. Die medizinaltechnische Werkzeughaltevorrichtung kann dabei als ganze Vorrichtung, daher ohne ein weiteres Zerlegen, sterilisiert werden. Ein Vorteil der erfindungsgemässen Werkzeughaltevorrichtung ist die Tatsache, dass das Auslösemoment durch die Reinigung nicht beeinflusst wird. Zudem kann die Sterilisation auch im zusammengebauten, einsatzbereiten Zustand des chirurgischen Instrumentes erfolgen.

Ein weiterer Vorteil der erfindungsgemässen Werkzeughaltevorrichtung ist die Tatsache, dass das Auslösemoment über eine Federvorspannung einstellbar ist, und dass die Rollfläche bzw. die Vertiefungen für die Kugeln sehr weit aussen angeordnet sind, sodass auch bei relativ kleinem Aussendurchmesser der Werkzeughaltevorrichtung ein grosses Drehmoment übertragbar ist bzw. ein grosses Auslösemoment einstellbar ist.

Ein weiterer Vorteil der erfindungsgemässen Werkzeughaltevorrichtung ist die Tatsache, dass die angreifenden Kräfte auf viele Kugeln verteilbar sind, was zu einem sehr geringen Verschleiss führt. Zudem ist das Auslösemoment im wesentlichen nicht von der newtonschen Reibung abhängig, da die Rollreibung im wesentlichen für das Auslösemoment bestimmend ist.

Die Erfindung wird weiter an Hand mehrerer Ausführungsbeispiele im Detail beschrieben. Es zeigen:
- Fig. 1: eine Seitenansicht einer medizinaltechnischen Werkzeughaltevorrichtung eingebaut in ein medizinaltechnisches Instrument;
- Fig. 2a: ein Längsschnitt durch das zweite Gehäuseteil;
- Fig. 2b: eine perspektivische Ansicht des zweiten Gehäuseteils;
- Fig. 3a: ein Längsschnitt durch den Kugelmitnehmer;
- Fig. 3b: eine Detailaspekt A des Kugelmitnehmers;
- Fig. 4: eine Seitenansicht des Kraftumlenkelementes;
- Fig. 5a: ein Längsschnitt durch die Einstellmutter;
- Fig. 5b: eine perspektivische Ansicht einer weiteren Ausführungsform einer Einstellmutter.

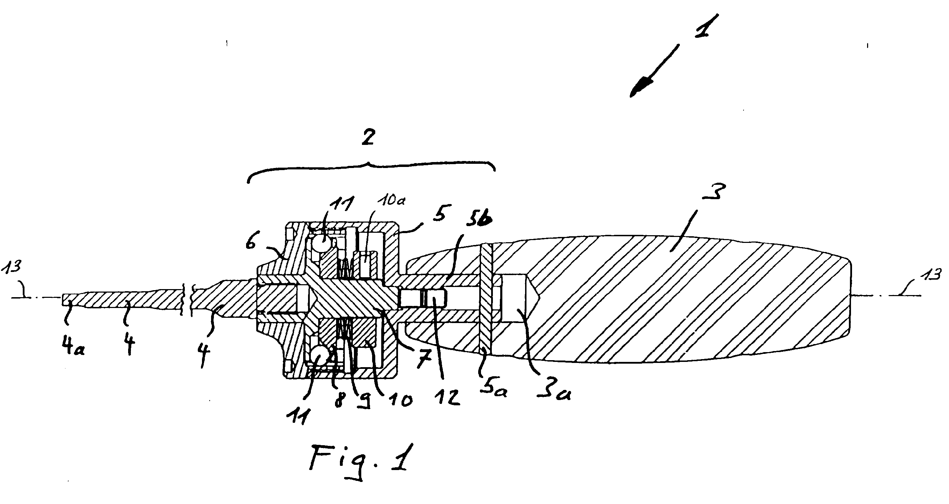

Fig. 1 zeigt die Seitenansicht eines chirugischen Instrumentes 1 umfassend eine medizinaltechnische Werkzeughaltevorrichtung 2 mit Drehmomentbegrenzung. Die Werkzeughaltevorrichtung 2 umfasst eine Gehäuse 5,6, welches aus zwei miteinander verschraubten Teilgehäusen, einem ersten Teilehäuse 5 sowie einem zweiten Teilgehäuse 6 besteht. Im Gehäuse 5,6 ist ein Kugelmitnehmer 7 um eine Drehachse 13 drehbar gelagert. Ein Werkzeug 4 mit Sechskant 4a ist lösbar mit dem Kugelmitnehmer 7 verbunden. Ein Handgriff 3 ist mittels eines Zylinderstifts 5a lösbar mit einem sich in Verlaufsrichtung der Drehachse 13 erstreckenden Fortsatzes 5b des ersten Teilgehäuses 5 verbunden.

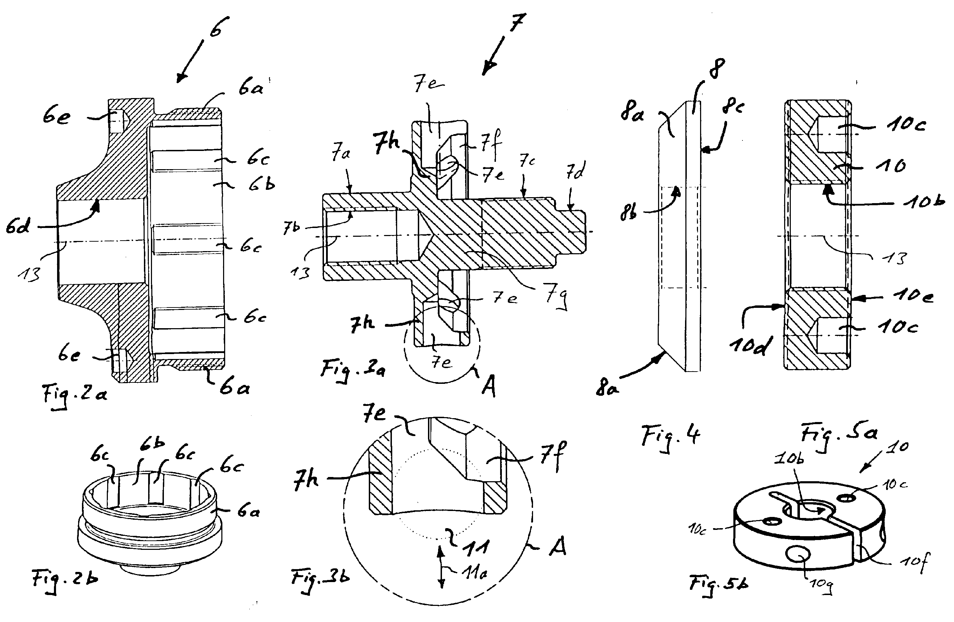

Die Werkzeughaltevorrichtung 2 ist derart ausgestaltet, dass beim Überschreiten eines bestimmten, am Handgriff 3 anliegenden Drehmomentes dieses nicht mehr auf des Werkzeug 4 übertragen wird. Um diese Funktion zu erfüllen sind in Verlaufsrichtung der Welle 13 hintereinander das zweite Teilgehäuse 6, der Kugelmitnehmer 7, die Kugeln 11, das Kraftumlenkelement 8, die Feder 9 sowie die Einstellmutter 10 angeordnet. Diese Komponenten sind, mit Ausnahme der Feder 9, in den Figuren 2a, 3a, 4 und 5 in derselben Reihenfolge angeordnet und werden mit Hilfe dieser Figuren im Detail erläutert.

Das zweite Teilgehäuse 6 umfasst ein Aussengewinde 6a, an welchem das erste Teilgehäuse 5 befestigbar ist. Das zweite Teilgehäuse 6 umfasst zudem eine im wesentlichen kozentrisch zur Drehachse 13 verlaufende, kreisförmig ausgestaltete Rollfläche 6b, welche in regelmässigen Abständen von einer Vertiefung 6c unterbrochen ist. Das zweite Teilgehäuse 6 umfasst zudem eine in Richtung der Drehachse 13 verlaufende Bohrung 6d sowie zwei Ansenkungen 6e. Fig. 2b zeigt dasselbe zweite Teilgehäuse 6 in einer perspektivischen Ansicht.

Fig. 3a zeigt einen Längsschnitt durch den Kugelmitnehmer 7. Dieser umfasst einen in Richtung der Drehachse 13 verlaufendes Nabenteil 7g sowie ein dazu senkrecht bzw. radial zur Drehachse 13 verlaufendes Flanschteil 7h. Das Nabenteil 7g weist ein Gleitfläche 7d auf, welche, wie in Fig. 1 dargestellt, zusammen mit dem ersten Teilgehäuse 5 ein Lager ausbildet. Das Nabenteil 7g weist zudem eine Gleitfläche 7a auf, welche zusammen mit der Bohrung 6d des zweiten Teilgehäuses 6 ein Lager ausbildet, sodass das Nabenteil 7g in einer durch das Gehäuse 5,6 definierten Drehachse 13 gelagert ist. Das Nabenteil 7g umfasst zudem eine Bohrung mit Innengewinde 7b zum Befestigen des Werkzeuges 4. Weiter umfasst das Nabenteil 7g ein Aussengewinde 7c, an welchem eine Einstellmutter 10 befestigbar ist. Das Flanschteil 7h umfasst eine in Umfangsrichtung verlaufende Mitnahmeführung 7f sowie eine Mehrzahl in Umfangsrichtung regelmässig beabstandet angeordnete, bezüglich der Drehachse 13 in radialer Richtung verlaufende Bohrungen bzw. Führungen 7e. Fig. 3b zeigt den Ausschnitt A gemäss Fig. 3a im Detail. Die im Flanschteil 7h angeordnete Führung 7e weist in radialer Richtung eine Durchbrechung auf, in welcher eine Kugel 11 in Richtung 11a verschiebbar angeordnet ist. In jeder Führung 7e ist eine Kugel 11 angeordnet.

Das in Fig. 4 dargestellte Kraftumlenktungsteil 8 mit Bohrung 8b ist auf dem Nabenteil 7g verschiebbar gelagert. Die in Fig. 5a dargestellte Einstellmutter 10 weist ein Innengewinde 10b auf, welches derart entsprechend dem Aussengewinde 7c des Nabenteils 7g ausgestaltet ist, dass die Einstellmutter 10 über das Aussengewinde 7c lösbar mit dem Kugelmitnehmer 7 verbindbar ist. Durch ein entsprechendes Drehen der Einstellmutter 10 kann deren Lage bezüglich dem Kugelmitnehmer 7 in Verlaufsrichtung der Drehachse 13 verstellt werden. Zwischen der Stirnseite 10d der Einstellmutter 10 und der Stirnseite 8c der Kraftumlenkelementes 8 ist, wie in Fig. 1 dargestellt, eine in Richtung der Drehachse 13 wirkende Feder 9 angeordnet. Diese Feder 9 kann beispielsweise als eine Sprialfeder, eine Tellerfeder, oder eine aus dem vollen gefräste Feder ausgestaltet sein. Im Gegensatz zur Ausführungform gemäss Fig. 5a weist die in Fig. 5b dargestellte Einstellmutter 10 einen Spalt 10f auf sowie eine senkrecht zu den Begrenzungsflächen des Spaltes 10f verlaufende Bohrung 10g, welche teilweise ein Innengewinde aufweist. Die Einstellmutter 10 ist mit einer nicht dargestellten, durch die Bohrung 10g verlaufenden Schraube auf dem Kugelmitnehmer 7 fixierbar.

Wie aus Fig. 1 ersichtlich ist das Kraftumlenkelement 8 federbelastet vorgespannt, wobei die an den Kugeln 11 anliegende Stirnseite 8a derart ausgestaltet ist, dass die Stirnseite 8a eine bezüglich der Drehachse 13 in radialer Richtung wirkende Kraft auf die Kugeln 11 ausübt, sodass diese gegen die Rollfäche 6b oder in die Vertiefungen 6c gedrückt werden. Das Kraftumlenkelement 8 erlaubt somit die in Verlaufsrichtung der Drehachse 13 wirkende Kraft der Feder 9 in eine radial auf die Kugeln 11 wirkende Kraft umzulenken.

In einer Grundstellung befindet sich in jeder Vertiefung 6c eine Kugel 11. Übersteigt das an der Werkzeughaltevorrichtung 2 angreifende Drehmoment eine vorbestimmbare Grösse, wo wird die Kugel 11 in Verschieberichtung 11a aus der Vertiefung 6c bewegt und die Kugeln 11 rollen über die Rollfäche 6b. Dadurch wird das maximal übertragbare Drehmoment begrenzt. Das maximal übertragbare Drehmoment ist unter anderem Abhängig von der durch die Feder 9 auf das Kraftumlenkelement 8 bewirkte Kraft. Diese Kraft ist durch ein Verschieben der Einstellmutter 10 in Richtung der Drehachse 13 einstellbar. Die Einstellmutter 10 weist auf der der Feder 9 abgewandten Stirnseite 10e zwei Bohrungen 10c auf, in welche mit einem entsprechend ausgeformten Schlüssel eingreifbar ist, um die Einstellmutter 10 anzuziehen oder zu lösen. Die auf das Kraftumlenkelement 8 bewirkte Kraft kann auch durch eine entsprechende Wahl der Feder 9 beeinflusst werden. Es kann somit ein Satz Federn 9 mit unterschiedlichen Elastizitätsmodulen vorgesehen sein, wobei abhängig vom gewünschten, maximal übertragbaren Drehmoment die entsprechende Feder zwischen dem Kraftumlenkelement 8 und der Einstellmutter 10 angeordnet wird.

Es kann sich als vorteilhaft erweisen die sich innerhalb des Gehäuses 5,6 befindlichen, beweglichen Teile mit einem sterilisierbaren Öl zu schmieren, um den Verschleiss zusätzlich zu reduzieren oder die Reproduzierbarkeit des maximal übertragbaren Drehmomentes bzw. des Auslösemomentes zu erhöhen.

## Patentansprüche

1. Medizinaltechnische Werkzeughaltevorrichtung (2) mit Drehmomentbegrenzung, umfassend ein Gehäuse (5, 6) und einen darin um eine Drehachse (13) drehbar gelagerten Kugelmitnehmer (7), wobei der Kugelmitnehmer (7) ein in Richtung der Drehachse (13) verlaufendes Nabenteil (7g) sowie ein Flanschteil (7h) umfasst, wobei im Flanschteil (7h) eine Mehrzahl in Umfangsrichtung beabstandete, im wesentlichen radial zur Drehachse (13) verlaufende Führungen (7e) zur Aufnahme einer Kugel (11) angeordnet sind, und wobei das Gehäuse (5, 6) eine im wesentlichen konzentrisch zur Drehachse (13) verlaufende, zur Drehachse (13) hin ausgerichtete Rollfläche (6b) mit in Umfangsrichtung beabstandet angeordneten Vertiefungen (6c) aufweist, und wobei ein Kraftumlenkelement (8) am Nabenteil (7g) in Richtung der Drehachse (13) verschiebbar und federbelastet gelagert ist, und wobei zwischen dem Flanschteil (7h) und dem Kraftumlenkelement (8) zumindest eine Kugel (11) angeordnet ist, und wobei die zum Flanschteil (7h) hin ausgerichtete Stirnseite (8a) des Kraftumlenkteils (8) derart ausgestaltet ist, dass die an der Stirnseite (8a) anliegende Kugel (11) eine bezüglich der Drehachse (13) radial wirkende Kraft erfährt, um die Kugel (11) gegen die Rollfläche (6b) oder in die Vertiefung (6c) zu drücken.

2. Medizinaltechnische Werkzeughaltevorrichtung nach Anspruch 1, dadurch gekennzeichnet, dass dieselbe Anzahl Führungen (7e) und Vertiefungen (6c) vorgesehen sind, und dass diese in Umfangsrichtung regelmässig beabstandet angeordnet sind.

3. Medizinaltechnische Werkzeughaltevorrichtung nach einem der vorhergehenden Ansprüche, dadurch gekennzeichnet, dass am Nabenteil (7g) eine Einstellmutter (10) befestigbar ist, wobei zwischen dem Flanschteil (7h) und der Einstellmutter (10) eine Feder (9) angeordnet ist.

4. Medizinaltechnische Werkzeughaltevorrichtung nach Anspruch 3, dadurch gekennzeichnet, dass das Nabenteil (7g) ein Aussengewinde (7c) aufweist, dass die Einstellmutter (10) ein dem Aussengewinde (7c) angepasstes Innengewinde (10b) aufweist, und dass die Einstellmutter (10) mit deren Innengewinde (10b) drehbar auf dem Aussengewinde (7c) des Nabenteils (7g) befestigt ist, um die Lage der Einstellmutter (10) in Verlaufsrichtung der Drehachse (13) zu verstellen.

5. Medizinaltechnische Werkzeughaltevorrichtung nach einem der Ansprüche 3 oder 4, dadurch gekennzeichnet, dass die Einstellschraube (10) eine bezüglich der Drehachse (13) in radialer Richtung verlaufende Bohrung zur Aufnahme einer Feststellschraube (10a) aufweist.

6. Medizinaltechnische Werkzeughaltevorrichtung nach einem der Ansprüche 3 bis 5, dadurch gekennzeichnet, dass die Einstellschraube (10) auf der der Feder (9) abgewandten Stirnseite (10e) zumindest eine Ausnehmung (10c) aufweist.

7. Medizinaltechnische Werkzeughaltevorrichtung nach einem der Ansprüche 3 bis 6, dadurch gekennzeichnet, dass die Feder (7) als Tellerfeder ausgestaltet ist.

8. Medizinaltechnische Werkzeughaltevorrichtung nach einem der vorhergehenden Ansprüche, dadurch gekennzeichnet, dass das Gehäuse (5, 6) zwei lösbar verbindbare Teilgehäuse (5, 6) umfasst.

9. Medizinaltechnische Werkzeughaltevorrichtung nach einem der vorhergehenden Ansprüche, dadurch gekennzeichnet, dass ein Werkzeug (4) mit dem Kugelmitnehmer (7) und ein Handgriff (3) mit dem Gehäuse (5, 6) verbindbar ist, oder dass ein Handgriff (3) mit dem Kugelmitnehmer (7) und ein Werkzeug (4) mit dem Gehäuse (5, 6) verbindbar ist.

10. Chirurgisches Instrument umfassend eine Werkzeughaltevorrichtung nach einem der vorhergehenden Ansprüche.
